# EUROPÄISCHE PATENTSCHRIFT

(11) **EP 3 585 456 B1**
(45) Veröffentlichungstag und Bekanntmachung des Hinweises auf die Patenterteilung: **05.05.2021**
(21) Anmeldenummer: 18707320.0
(22) Anmeldetag: 20.02.2018
(51) Int. Cl.: A61M 1/36, A61M 39/28

(54) **VORRICHTUNG ZUM ABKLEMMEN EINER SCHLAUCHLEITUNG UND MEDIZINISCHE BEHANDLUNGSVORRICHTUNG MIT EINER VORRICHTUNG ZUM ABKLEMMEN EINER SCHLAUCHLEITUNG UND VERFAHREN ZUR ÜBERWACHUNG EINER VORRICHTUNG ZUM ABKLEMMEN EINER SCHLAUCHLEITUNG**
DEVICE FOR CLAMPING A HOSE LINE, MEDICAL TREATMENT DEVICE WITH A DEVICE FOR CLAMPING A HOSE LINE, AND METHOD FOR MONITORING A DEVICE FOR CLAMPING A HOSE LINE
DISPOSITIF POUR CLAMPER UNE TUBULURE ET DISPOSITIF DE TRAITEMENT MÉDICAL AYANT UN DISPOSITIF POUR CLAMPER UNE TUBULURE ET PROCÉDÉ POUR SURVEILLER UN DISPOSITIF POUR CLAMPER UNE TUBULURE

(30) Priorität: 23.02.2017 DE 102017001744
(43) Veröffentlichungstag der Anmeldung: 01.01.2020
(73) Patentinhaber: Fresenius Medical Care Deutschland GmbH, 61352 Bad Homburg v.d.H (DE)
(72) Erfinder: GRÖBER, Tobias, 63150 Heusenstamm (DE)
(74) Vertreter: Oppermann, Frank
(86) Internationale Anmeldenummer: PCT/EP2018/054182
(87) Internationale Veröffentlichungsnummer: WO 2018/153875

(56) Entgegenhaltungen:
- EP-A1- 2 517 753
- DE-A1-102015 213 206
- US-A1- 2001 019 117
- US-A1- 2010 188 667

## Beschreibung

Die Erfindung betrifft eine Vorrichtung zum Abklemmen einer Schlauchleitung, die über einen Klemmkörper, eine elektromagnetische Betätigungseinheit für den Klemmkörper und eine die Position des Klemmkörpers überwachende Überwachungseinrichtung verfügt, wobei die Betätigungseinheit eine Spule zur Erzeugung eines Magnetfeldes aufweist. Darüber hinaus betrifft die Erfindung eine medizinische Behandlungsvorrichtung, insbesondere Vorrichtung zur extrakorporalen Blutbehandlung, mit einer Vorrichtung zum Abklemmen einer Schlauchleitung sowie ein Verfahren zur Überwachung einer Vorrichtung zum Abklemmen einer Schlauchleitung.

Der extrakorporale Blutkreislauf der bekannten Blutbehandlungsvorrichtungen weist eine arterielle Schlauchleitung auf, die zu der Blutkammer des Blutbehandlungselements, z.B. einem Dialysator führt, und eine venöse Schlauchleitung auf, die von der Blutkammer abgeht. Die Schlauchleitungen der extrakorporalen Blutbehandlungsvorrichtung werden im Allgemeinen als zur einmaligen Verwendung bestimmtes Schlauchset (Disposable) bereitgestellt. Die Blutbehandlungsvorrichtungen verfügen über eine Blutpumpe, die im Allgemeinen stromauf der Blutkammer des Blutbehandlungselements angeordnet ist, um einen ausreichenden Blutfluss im extrakorporalen Blutkreislauf sicher zu stellen.

Zur Unterbrechung des Blutflusses verfügen die Blutbehandlungsvorrichtungen über automatische Schlauchklemmen, beispielsweise eine venöse Schlauchklemme zur Unterbrechung des Blutflusses in der venösen Blutleitung. Die bekannten automatischen Schlauchklemmen weisen einen axial verschiebbaren Klemmkörper auf, der federnd auf eine Anlagefläche vorgespannt ist. Der Klemmkörper wird von einer elektromagnetischen Betätigungseinheit betätigt, die eine Spule zur Erzeugung eines Magnetfeldes aufweist, das eine Kraft auf den Klemmkörper ausübt.

Beim Aufrüsten der Blutbehandlungsvorrichtungen wird die Schlauchleitung in die Schlauchklemme zwischen Klemmkörper und Anlagefläche eingelegt, so dass die Schlauchleitung abgeklemmt ist. Für die Durchführung der Blutbehandlung wird die Schlauchklemme automatisch geöffnet, so dass Flüssigkeit durch die Schlauchleitung strömen kann.

Die venöse Schlauchklemme zur Unterbrechung des Blutflusses in der venösen Blutleitung ist für einen sicheren Betrieb der Blutbehandlungsvorrichtungen von großer Bedeutung. In einem Störfall wird die venöse Schlauchklemme automatisch geschlossen, so dass der Blutfluss unterbrochen ist. Ansonsten könnte der Patient verbluten. Dies setzt aber voraus, dass beim Aufrüsten der Blutbehandlungsvorrichtung die Schlauchleitung in die Schlauchklemme auch richtig eingelegt worden ist. Ansonsten wäre die Schlauchklemme wirkungslos.

Die zur Blutbehandlung verwendeten Schlauchsets umfassen im Allgemeinen eine relativ große Anzahl von Schlauchleitungen. Daher ist eine gewisse Erfahrung notwendig, um das Schlauchset richtig in die Blutbehandlungsvorrichtung einzulegen. Zwar kann eine nicht richtig eingelegte Schlauchleitung bei einem der Blutbehandlung vorausgehenden Druckhaltetest erkannt werden, mit dem die Integrität des Dialysators geprüft wird. Dieser Druckhaltetest erlaubt aber nicht unter sämtlichen Betriebsbedingungen immer eine sichere Erkennung, ob die Schlauchleitung richtig in die Schlauchklemme eingelegt ist. Zum Stand der Technik gehören Vorrichtungen zum Abklemmen von Schlauchleitungen, die über einen Schalter verfügen, der von der Schlauchleitung geschlossen wird, wenn die Schlauchleitung in die Schlauchklemme eingelegt wird. Nachteilig ist, dass für die Überwachung der Schlauchleitung ein zusätzliches mechanisches Bauteil erforderlich ist. Dadurch werden die Herstellungskosten erhöht. Mit dem Einbau eines Schalters ergeben sich auch konstruktionsbedingt vorspringende Teile und Gehäusespalte, wodurch die Reinigung erschwert wird. Des Weiteren erlaubt die Integration eines Schalters in die Schlauchklemme nur die Erkennung von zwei Betriebszuständen, ob eine Schlauchleitung in die Schlauchklemme eingelegt ist oder nicht eingelegt ist

Eine bekannte Klemmvorrichtung ist z.B. in US2010188667 offenbart.

Der Erfindung liegt die Aufgabe zugrunde, eine Vorrichtung zum Abklemmen einer Schlauchleitung zu schaffen, die über eine Überwachungseinrichtung verfügt, die ohne zusätzliche mechanische Bauteile zumindest sicher erkennen kann, ob eine Schlauchleitung in die Schlauchklemme eingelegt ist oder nicht eingelegt ist.

Der Erfindung liegt weiterhin die Aufgabe zugrunde, eine Vorrichtung zum Abklemmen einer Schlauchleitung zu schaffen, die über eine Überwachungseinrichtung verfügt, die ohne zusätzliche mechanische Bauteile sicher erkennen kann, ob die Vorrichtung zum Abklemmen einer Schlauchleitung frei von elektrischen Defekten, wie beispielsweise Spulenbruch aufgrund von thermischer Belastung, ist.

Ferner liegt der Erfindung die Aufgabe zugrunde, eine Vorrichtung zum Abklemmen einer Schlauchleitung zu schaffen, die über eine Überwachungseinrichtung verfügt, die ohne zusätzliche mechanische Bauteile sicher erkennen kann, ob die Vorrichtung zum Abklemmen einer Schlauchleitung frei von mechanischen Blockaden ist, welche das Öffnen/Schließen des Ventils behindern, beispielsweise eine mechanische Verklemmung oder ein vom Nutzer versehentlich angebrachter Fremdkörper.

Die Lösung dieser Aufgaben erfolgt erfindungsgemäß mit den Merkmalen der unabhängigen Patentansprüche. Vorteilhafte Ausführungsformen der Erfindung sind Gegenstand der abhängigen Ansprüche.

Die Erfindung beruht auf der Überwachung einer von der Stellung des Klemmkörpers abhängigen elektrischen Eigenschaft der Spule.

Die Überwachungseinrichtung der erfindungsgemäßen Vorrichtung zum Abklemmen einer Schlauchleitung verfügt über eine Messeinrichtung zum Messen einer von der Stellung des Klemmkörpers abhängigen elektrischen Eigenschaft der Spule und eine Auswerteeinrichtung zum Bestimmen der Position des Klemmkörpers auf der Grundlage der elektrischen Eigenschaft der Spule. Da nur eine elektrische Eigenschaft der Spule überwacht wird, ist für die Überwachung ein zusätzliches mechanisches Bauteil nicht erforderlich. Daraus ergeben sich geringere Herstellungskosten. Darüber hinaus wird die Reinigung der Vorrichtung nicht durch vorspringende Teile oder Spalte eines zusätzlichen mechanischen Bauteils erschwert.

Eine Ausführungsform der Erfindung sieht als eine von der Stellung des Klemmkörpers abhängige elektrische Eigenschaft die Induktivität der Spule vor. Die Induktivität der Spule wird von der räumlichen Anordnung von Spule und Klemmkörper bestimmt. Zum Messen der Induktivität der Spule ist eine Messeinrichtung vorgesehen. Für die Erkennung der Position des Klemmkörpers brauchen für die Induktivität der Spule keine absoluten Werte bestimmt werden, sondern die Bestimmung relativer Werte kann ausreichend sein. Insofern erweist sich die Auswertung des Messergebnisses als einfach.

Wenn in diesem Zusammenhang von einem Klemmkörper oder einer Spule die Rede ist, ist darunter nicht zu verstehen, dass die Vorrichtung zum Abklemmen einer Schlauchleitung nur über einen einzigen Klemmkörper und/oder nur über eine einzige Spule verfügt. Die Vorrichtung kann auch über mehrerer Klemmkörper verfügen, die auf die Schlauchleitung von einer und/oder mehreren Seiten einwirken. Ein Klemmkörper kann durch das magnetische Feld einer oder mehrerer Spulen betätigt werden.

Der Klemmkörper kann zum Verklemmen der Schlauchleitung eine axiale und/oder rotatorische Bewegung ausführen. Eine bevorzugte Ausführungsform sieht als Klemmkörper einen axial beweglichen Anker und als Spule eine Hohlspule vor, in die der Klemmkörper eintaucht. Die Induktivität der Spule nimmt mit zunehmender Eintauchtiefe des als Anker ausgebildeten Klemmkörpers zu. Daher ist die Induktivität eine von der Position des Klemmkörpers abhängige elektrische Größe.

Der Klemmkörper ist vorzugsweise in eine die Schlauchleitung abklemmende Position federnd vorgespannt, so dass bei stromloser Spule die Schlauchleitung abgeklemmt ist. Daher ist die Grundstellung der Schlauchklemme eine die Schlauchleitung abklemmende Stellung. Ein Ausfall der Stromversorgung kann daher nicht zu einem unbeabsichtigten Öffnen der Schlauchklemme führen.

Erfindungsgemäß weist die Überwachungseinrichtung eine Auswerteeinrichtung auf, die eine Vergleichseinrichtung aufweist, die derart ausgebildet ist, dass die elektrische Eigenschaft der Spule mit mindestens einem vorgegebenen Grenzwert verglichen wird. Die Auswerteeinrichtung ist derart ausgebildet, dass bei Überschreiten oder Unterschreiten des mindestens einen Grenzwertes auf die von dem Klemmkörper eingenommene Position geschlossen wird. Bei dieser Ausführungsform ist die Bestimmung absoluter Werte für die elektrische Eigenschaft der Spule nicht erforderlich. Zur Auswertung der Messergebnisse reicht aus, die Messwerte mit einem oder mehreren Grenzwerten zu verglichen, die für eine bestimmte Position des Klemmkörpers charakteristisch sind. Beispielsweise kann geprüft werden, ob die Messwerte innerhalb oder außerhalb eines für die jeweilige Position des Klemmkörpers charakteristischen Grenzwertfensters liegen. Diese Grenzwerte können empirisch ermittelt und in einem Speicher der Auswerteeinrichtung gespeichert werden.

Die erfindungsgemäße Vorrichtung zum Abklemmen einer Schlauchleitung erlaubt die Erkennung mehrerer Betriebszustände, in denen der Klemmkörper eine bestimmte Position einnimmt.

Die Vergleichseinrichtung ist derart ausgebildet, dass auf eine erste Position geschlossen wird, in der der Ventilkörper eine vollständig geöffnete Position einnimmt, in der eine Schlauchleitung in die Vorrichtung zum Abklemmen der Schlauchleitung eingelegt werden kann, wenn die elektrische Eigenschaft der Spule kleiner als ein erster oberer Grenzwert und/oder größer als ein erster unterer Grenzwert ist.

Darüber hinaus ist die Vergleichseinrichtung derart ausgebildet, dass auf eine zweite Position geschlossen wird, in der der Ventilkörper eine Position einnimmt, in der eine in die Vorrichtung zum Abklemmen des Ventilkörpers eingelegte Schlauchleitung abgeklemmt wird, wenn die elektrische Eigenschaft der Spule kleiner als ein zweiter oberer Grenzwert und/oder größer als ein zweiter unterer Grenzwert ist.

Des weiteren ist die Vergleichseinrichtung derart ausgebildet, dass auf eine dritte Position geschlossen wird, in der der Ventilkörper eine vollständig geschlossene Position einnimmt, in der eine Schlauchleitung in die Vorrichtung zum Abklemmen der Schlauchleitung nicht eingelegt ist, wenn die elektrische Eigenschaft der Spule kleiner als ein dritter oberer Grenzwert und/oder größer als ein dritter unterer Grenzwert ist.

Der erste bzw. zweite bzw. dritte obere und untere Grenzwert definieren jeweils ein Grenzwertfenster, in dem der Messwert für die Induktivität liegt, wenn einer der drei Betriebszustände vorliegt. Dabei kann der erste, obere und untere Grenzwert größer als der zweite, obere und untere Grenzwert und der zweite, obere und untere Grenzwert größer als der dritte, obere und untere Grenzwert sein, wenn die von der Position des Klemmkörpers abhängige Größe die Induktivität der Spule ist.

Zur Vereinfachung der Messung der Induktivität sieht eine weitere besonders bevorzugte Ausführungsform vor, dass die Messeinrichtung eine Spannungsquelle zur Erzeugung einer Testspannung aufweist, die an die Spule angelegt wird, so dass durch die Spule ein Strom fließt, und einen Spannungsmesser aufweist, der die zeitliche Änderung der an der Spule abfallenden Spannung misst. Die Auswerteeinrichtung ist bei dieser Ausführungsform derart ausgebildet, dass nicht absolute Werte für die Induktivität ermittelt werden, sondern der zeitliche Verlauf des Spannungsabfalls an der Spule als eine mit der Induktivität der Spule korrelierende Größe ausgewertet wird.

Die Vergleichseinrichtung der Auswerteeinrichtung ist vorzugsweise derart ausgebildet, dass die Abnahme der Spannung in einem vorgegebenen Zeitintervall mit mindestens einem Grenzwert verglichen wird.

Anstelle der Messung des Spannungsabfalls an der Spule ist es auch möglich, die Änderung des durch die Spule fließenden Stromes auszuwerten. Anstelle der Induktivität der Spule können auch andere elektrische Größen ausgewertet werden. Beispielsweise ist auch möglich, durch Parallelschaltung eines Kondensators mit der Spule einen elektrischen Schwingkreis zu bilden, dessen Resonanzfrequenz von der Induktivität der Spule, die von der Position des Klemmkörpers abhängig ist, bestimmt wird.

Anstelle der Messung mit einer Testspannung kann die Messung auch bei Schalten des Ventils (Öffnen/Schließen) mithilfe der Schaltspannung durchgeführt werden.

In einer weiteren Ausführungsform kann als elektrische Eigenschaft der Spule auch die Impedanz des Systems bestimmt werden. Hierzu wird als Testspannung eine Wechselspannung angelegt und der zeitliche Mittelwert des Systemwiderstands bestimmt.

Die Überwachungseinrichtung weist vorzugsweise eine Anzeigeeinrichtung auf, die derart ausgebildet ist, dass die von dem Klemmkörper eingenommene Position angezeigt wird. Die Anzeigeeinrichtung kann beispielsweise ein Display sein.

Weiterhin kann eine Alarmeinrichtung vorgesehen sein, die derart ausgebildet ist, dass ein Alarm gegeben wird, wenn der Klemmkörper eine bestimmte Position einnimmt, beispielsweise die Schlauchleitung nicht in die Vorrichtung eingelegt ist.

Die Überwachungseinrichtung kann auch ein Steuersignal erzeugen, wenn ein bestimmter Betriebszustand erkannt wird, beispielsweise die Schlauchleitung nicht in die Klemmvorrichtung eingelegt ist, so dass ein Eingriff in die Maschinensteuerung der Blutbehandlungsvorrichtung vorgenommen werden kann, beispielsweise die Blutpumpe gestoppt werden kann.

Die erfindungsgemäße Vorrichtung zur extrakorporalen Blutbehandlung kann eine oder mehrere der Vorrichtungen zum Abklemmen einer Schlauchleitung, insbesondere der venösen Schlauchleitung des extrakorporalen Blutkreislaufs, umfassen. Die Überwachungseinrichtung der Vorrichtung zum Abklemmen einer Schlauchleitung kann Bestandteil der zentralen Steuer- und Recheneinheit der Blutbehandlungsvorrichtung sein.

Nachfolgend wird ein Ausführungsbeispiel der Erfindung unter Bezugnahme auf die Zeichnungen im Einzelnen beschrieben.

Es zeigen:
- Fig. 1: in vereinfachter schematischer Darstellung eine erfindungsgemäße Vorrichtung zur extrakorporalen Blutbehandlung, die über eine erfindungsgemäße Vorrichtung zum Abklemmen einer Schlauchleitung verfügt,
- Fig. 2: die erfindungsgemäße Vorrichtung zum Abklemmen einer Schlauchleitung in vereinfachter schematischer Darstellung,
- Fig. 3: eine Schaltung zur Bestimmung der Induktivität der Spule der Betätigungseinheit für den Klemmkörper der Vorrichtung zum Abklemmen einer Schlauchleitung,
- Fig. 4A und 4B: den zeitlichen Verlauf der Testspannung und des Spannungsabfalls an der Spule für unterschiedliche Induktivitäten,
- Fig. 5A und 5B: den zeitlichen Verlauf der Testspannung und des Spannungsabfalls an der Spule in einem Zeitintervall, in dem die Testspannung auf einen vorgegebenen Grenzwert abgefallen ist,
- Fig. 6: die Vorrichtung zum Abklemmen einer Schlauchleitung in einem ersten Betriebszustand und den zugehörigen zeitlichen Verlauf des an der Spule gemessenen Spanungsabfalls,
- Fig. 7: die Vorrichtung zum Abklemmen einer Schlauchleitung in einem zweiten Betriebszustand und den zugehörigen zeitlichen Verlauf des Spanungsabfalls und
- Fig. 8: die Vorrichtung zum Abklemmen einer Schlauchleitung in einem dritten Betriebszustand und den zugehörigen zeitlichen Verlauf des an der Spule gemessenen Spanungsabfalls.

Bei dem vorliegenden Ausführungsbeispiel ist die medizinische Behandlungsvorrichtung eine extrakorporale Blutbehandlungsvorrichtung, insbesondere Hämodialysevorrichtung, die über einen extrakorporalen Blutkreislauf I mit einem Dialysator 1 verfügt, der durch eine semipermeable Membran 2 in eine Blutkammer 3 und eine Dialysierflüssigkeitskammer 4 unterteilt ist. Von einem Patienten führt ein arterielle Blutleitung 5, in der eine Blutpumpe 7 angeordnet ist, zu einem Einlass der Blutkammer 3, während von einem Auslass der Blutkammer eine venöse Blutleitung 6 zu dem Patienten führt. Die arterielle und venöse Blutleitung 5, 6 sind flexible Schlauchleitungen eines zur einmaligen Verwendung bestimmten Schlauchsets (Disposable).

Die frische Dialysierflüssigkeit wird in einer Dialysierflüssigkeitsquelle 10 bereitgestellt. Von der Dialysierflüssigkeitsquelle 10 führt eine Dialysierflüssigkeitszuführleitung 8 zu einem Einlass der Dialysierflüssigkeitskammer 4 des Dialysators 1, während eine Dialysierflüssigkeitsabführleitung 9 von einem Auslass der Dialysierflüssigkeitskammer 4 zu einem Abfluss 12 führt. In der Dialysierflüssigkeitsabführleitung 9 ist eine Dialysierflüssigkeitspumpe 30 angeordnet.

Die Ansteuerung der einzelnen Komponenten der Blutbehandlungsvorrichtung erfolgt mit einer zentralen Steuer- und Recheneinheit 13.

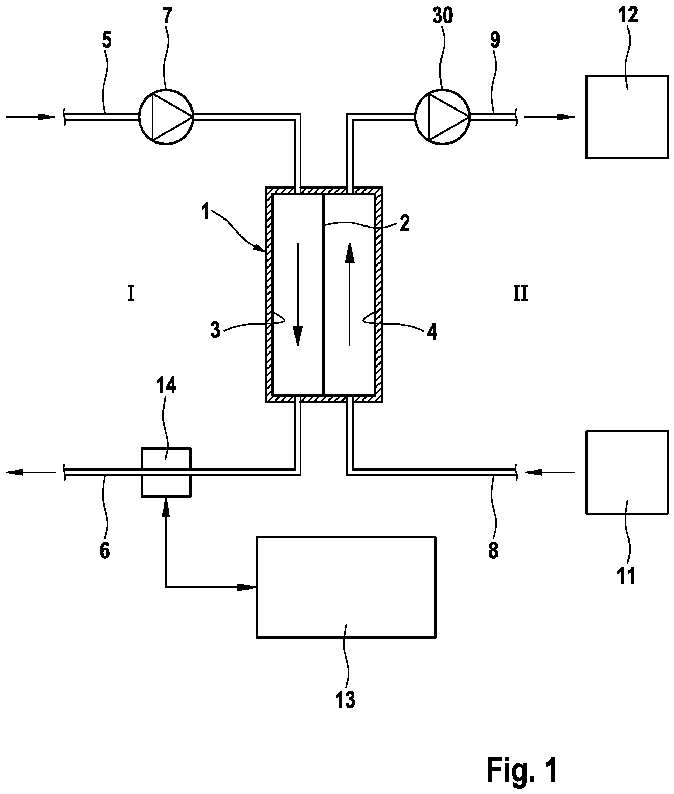

Zur Unterbrechung des Blutflusses zum Patienten ist eine in Fig. 1 nur schematisch dargestellte Vorrichtung 14 zum Abklemmen der venösen Schlauchleitung 6 vorgesehen. Beim Aufrüsten der Blutbehandlungsvorrichtung wird die venöse Schlauchleitung 6 in die Vorrichtung 14 zum Abklemmen der Schlauchleitung eingelegt. Die Vorrichtung 14 zum Abklemmen der Schlauchleitung wird nachfolgend im Einzelnen beschrieben.

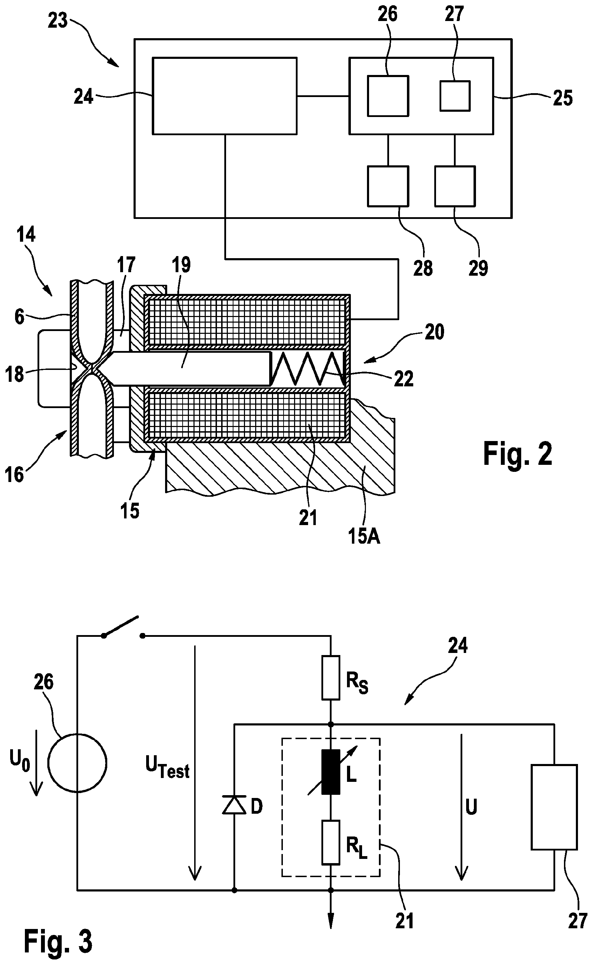

Fig. 2 zeigt die Vorrichtung 14 zum Abklemmen der Schlauchleitung in vereinfachter schematischer Darstellung. Die Vorrichtung weist einen Gehäusekörper 15 auf, der Bestandteil des Gehäuses 15A der Blutbehandlungsvorrichtung sein kann. Der Gehäusekörper 15 weist eine Aufnahme 16 für die Schlauchleitung 6 mit einer Auflagefläche 17 auf, auf der die in die Vorrichtung eingelegte Schlauchleitung aufliegt. Die Schlauchleitung 6 liegt zwischen einer Anlagefläche 18 des Gehäusekörpers 15 und einem Klemmkörper 19, der in dem Gehäusekörper 15 axial verschiebbar geführt ist. Der Klemmkörper 19 wird von einer elektromagnetischen Betätigungseinheit 20 betätigt, die als Linearmagnet (Hubmagnet) ausgebildet ist, der eine zylindrische Hohlspule 21 aufweist. Der Klemmkörper 19 ist als ein in die zylindrische Hohlspule 21 des Linearmagneten eintauchender Anker ausgebildet, der mit einer Druckfeder 22 in Richtung der Anlagefläche 18 federnd vorgespannt ist. Wenn durch die Hohlspule 21 ein Strom fließt, wird ein Magnetfeld erzeugt, das den Klemmkörper 19 entgegen der Federkraft in die Hohlspule 21 zurückzieht. Die elektromagnetische Betätigungseinheit 20 wird von der zentralen Steuer- und Recheneinheit 13 angesteuert.

Darüber hinaus verfügt die Vorrichtung 14 zum Abklemmen der Schlauchleitung über eine Überwachungseinrichtung 23, die eine Messeinrichtung 24 und eine Auswerteinrichtung 25 aufweist. Messeinrichtung 24 und Auswerteeinrichtung 25 sind in Fig. 2 nur schematisch dargestellt. Bei dem vorliegenden Ausführungsbeispiel ist die Messeinrichtung 24 als eine Messeinrichtung zum Messen der Induktivität der Spule ausgebildet.

Fig.3 zeigt eine Schaltung zur Verdeutlichung der Funktion der Messeinrichtung 24. Die Spule 21 ist in der Schaltung durch ein Ersatzschaltbild dargestellt. Die Spule 21 hat einen ohmschen Widerstand R_{L} und eine Induktivität L, die von der Eintauchtiefe d des Klemmkörpers 19 in die Spule abhängig ist.

Die Überwachungseinrichtung 23, insbesondere deren Auswerteeinrichtung 25, kann beispielsweise einen allgemeinen Prozessor, einen digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware-Komponenten aufweisen, um die einzelnen Verfahrensschritte zur Steuerung der Überwachungseinrichtung auszuführen. Auf den Hardware-Komponenten kann zur Durchführung der Verfahrensschritte ein Datenverarbeitungsprogramm (Software) laufen.

Die Überwachungseinrichtung 23 kann eine selbständige Baugruppe bilden, wobei die Messeinrichtung 24 und Auswerteeinrichtung 25 von den übrigen Komponenten der Blutbehandlungsvorrichtung unabhängig sind. Die Überwachungseinrichtung 23 oder Teile der Überwachungseinrichtung können aber auch Bestandteil der Komponenten der Blutbehandlungsvorrichtung sein. Beispielsweise kann die Auswerteeinrichtung 25 Bestandteil der zentralen Steuer- und Recheneinheit 13 der Blutbehandlungsvorrichtung sein.

Die Messeinrichtung 24 weist eine Spannungsquelle 26 zur Erzeugung einer Testspannung U_{Test} = U₀ und einen Spannungsmesser 27 zum Messen der an der Spule anliegenden Spannung U auf. Die Testspannung U_{Test} wird an eine Reihenschaltung eines Serienwiderstandes R_{S} und der Spule 21 angelegt. Folglich fließt durch die Spule 21 ein Strom, so dass ein Magnetfeld erzeugt wird. Die Höhe der Testspannung U_{Test} ist derart bemessen, dass der Klemmkörper 19 beim Test nicht bewegt wird. An der Spule 21 wird mit dem Spannungsmesser 27 die Spannung U gemessen, die von der Auswerteeinrichtung 25 ausgewertet wird.

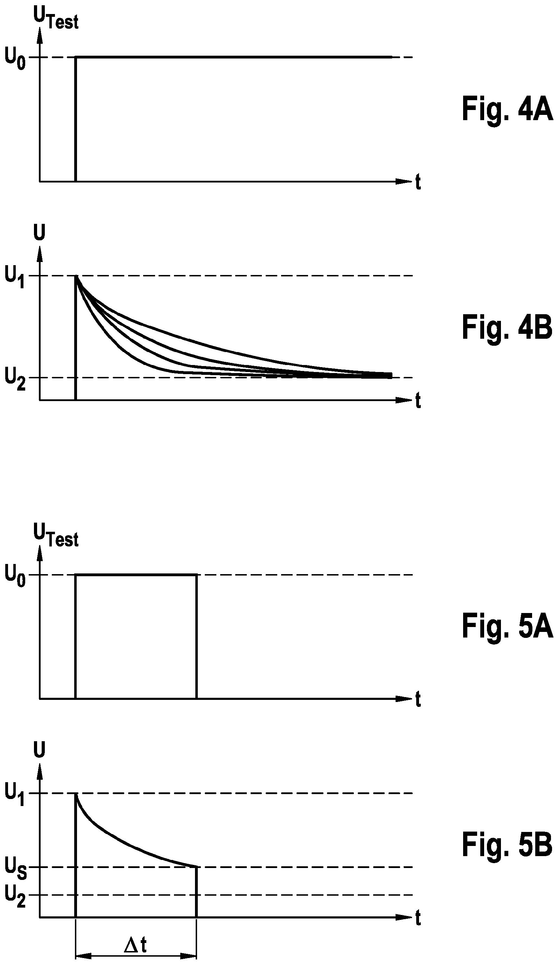

Die Figuren 4A und 4B zeigen die Testspannung U_{Test} = U₀ als Funktion der Zeit t und die Spannung U an der Spule 21. Die Spannung sinkt von einem Wert U₁ auf einen Wert U₂. Es zeigt sich eine exponentielle Abnahme der Spannung U, wobei die Zeitkonstante von der Induktivität L der Spule abhängig ist. Je größer die Induktivität L ist, desto geringer ist der Betrag, um den die Spannung U in einem vorgegebenen Zeitintervall von dem Wert U₁ abnimmt. Die Figuren 5A und 5B zeigen den zeitlichen Verlauf der Spannung U für die Testspannung U_{Test} = U₀. Das Zeitintervall Δt, in dem die Spannung auf den Grenzwert U_{S} abgenommen hat, ist von der Induktivität L der Spule 21 abhängig. Je größer das Zeitintervall Δt ist, desto größer ist die Induktivität L.

Die Auswerteeinrichtung 25 weist eine Vergleichseinrichtung 26 auf, die die gemessene Spannung U mit mehreren Grenzwerten vergleicht, die bei dem vorliegenden Ausführungsbeispiel drei Grenzwertfenster definieren. Die Grenzwerte werden aus einem Speicher 27 der Auswerteeinrichtung 25 ausgelesen.

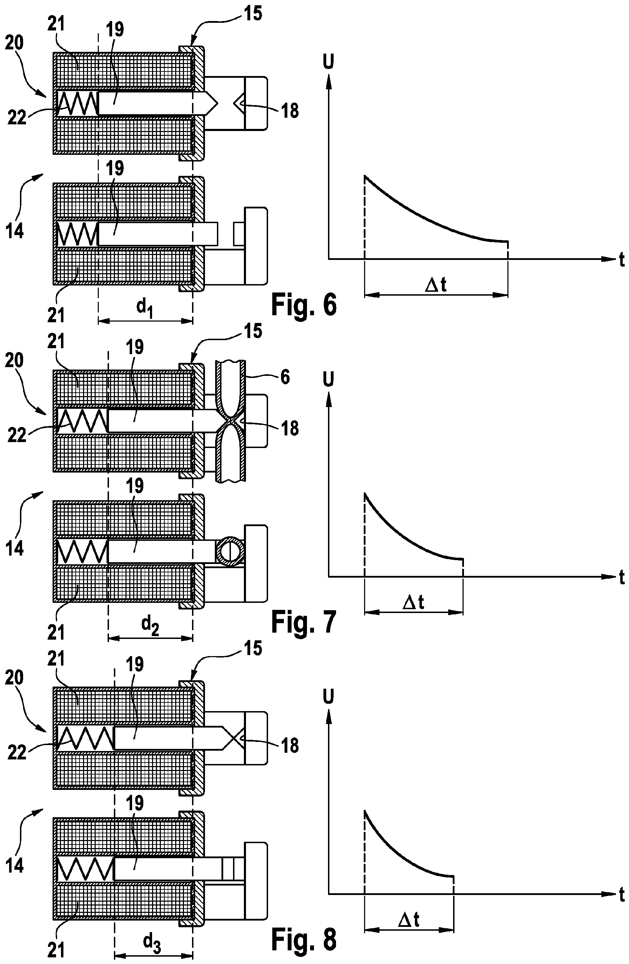

Die Vergleichseinrichtung 26 ist derart konfiguriert, dass auf einen ersten Betriebszustand geschlossen werden kann, in der der Klemmkörper 19 eine vollständig geöffnete Position einnimmt (Fig. 6). In dieser Position kann die Schlauchleitung 6 in die Vorrichtung 14 zum Abklemmen der Schlauchleitung eingelegt werden. Hierfür ist es erforderlich, dass der Klemmkörper 19 von Hand entgegen der Federkraft in die Feder 22 zurückgedrückt wird. In dieser Position ist die Eintauchtiefe d = d₁. Bei der Eintauchtiefe d₁ ist die Induktivität L = L₁. Es ergibt sich ein Zeitintervall, in dem die Spannung auf den Grenzwert U_{S} abgenommen hat, von Δt₁, das ein Maß für die Induktivität L ist. Die Auswerteeinrichtung 25 prüft, ob Δt₁ innerhalb eines ersten Grenzwertfensters liegt, das von einem ersten oberen Grenzwert und einem ersten unteren Grenzwert definiert wird. Wenn Δt₁ innerhalb des ersten Grenzwertfensters liegt, wird auf den ersten Betriebszustand geschlossen.

Die Vergleichseinrichtung 26 ist weiterhin derart konfiguriert, dass auf einen zweiten Betriebszustand geschlossen werden kann, in der der Klemmkörper 19 eine Position einnimmt, in der eine in die Vorrichtung 14 zum Abklemmen einer Schlauchleitung eingelegte Schlauchleitung 6 abgeklemmt wird (Fig. 7). In dieser Position ist die Eintauchtiefe d = d₂ (d₂ < d₁). Bei der Eintauchtiefe d₂ ist die Induktivität L = L₂. Es ergibt sich ein Zeitintervall, in dem die Spannung auf den Grenzwert U_{S} abgenommen hat, von Δt₂, das ein Maß für die Induktivität L ist. Die Auswerteeinrichtung 25 prüft, ob Δt₂ innerhalb eines zweiten Grenzwertfensters liegt, das von einem zweiten oberen Grenzwert und einem zweiten unteren Grenzwert definiert wird. Wenn Δt₂ innerhalb des zweiten Grenzwertfensters liegt, wird auf den zweiten Betriebszustand geschlossen.

Die Vergleichseinrichtung 26 kann auch auf einen dritten Betriebszustand schließen, in der der Klemmkörper 19 eine vollständig geschlossene Position einnimmt, in der eine Schlauchleitung 6 in die Vorrichtung 14 zum Abklemmen einer Schlauchleitung nicht eingelegt ist (Fig. 8). In dieser Position ist die Eintauchtiefe d = d₃ (d₃ < d₂). Bei der Eintauchtiefe d₃ ist die Induktivität L = L₃. Es ergibt sich ein Zeitintervall, in dem die Spannung auf den Grenzwert U_{S} abgenommen hat, von Δt₃, das ein Maß für die Induktivität L ist. Die Auswerteeinrichtung 25 prüft, ob Δt₃ innerhalb eines dritten Grenzwertfensters liegt, das von einem dritten oberen Grenzwert und einem dritten unteren Grenzwert definiert wird. Wenn Δt₂ innerhalb des dritten Grenzwertfensters liegt, wird auf den dritten Betriebszustand geschlossen.

Bei dem vorliegenden Ausführungsbeispiel werden Grenzwertfenster definiert, wobei überprüft wird, ob das Zeitintervall Δt innerhalb oder außerhalb der Grenzwertfenster liegt. Zur Ermittlung der unterschiedlichen Betriebszustände ist aber auch möglich, das Zeitintervall Δt nur mit einem oberen Grenzwert und einem unteren Grenzwert zu vergleichen. Wenn das Zeitintervall Δt oberhalb des oberen Grenzwertes liegt, wird auf den ersten Betriebszustand (Fig. 6) geschlossen, während auf den dritten Betriebszustand (Fig. 8) geschlossen wird, wenn das Zeitintervall Δt unterhalb des unteren Grenzwertes liegt. Wenn das Zeitintervall Δt zwischen dem oberen und unteren Grenzwert liegt, wird auf den zweiten Betriebszustand (Fig. 7) geschlossen.

Die Auswerteeinrichtung 25 erzeugt in Abhängigkeit von dem ermittelten Betriebszustand ein Signal, das dem jeweiligen Betriebszustand zugeordnet ist. Die Signale der Auswerteeinrichtung 25 werden von einer Anzeigeeinrichtung 28 empfangen, mit der der jeweilige Betriebszustand anzeigt wird, beispielsweise auf einem Display mit einem entsprechenden Symbol. Darüber hinaus können die Signale von einer Alarmeinrichtung 29 empfangen werden, die bei einem bestimmt Betriebszustand einen akustischen und/oder optischen und/oder taktilen Alarm gibt. Beispielsweise kann Alarm gegeben werden, wenn die Schlauchleitung 6 beim Starten der Blutbehandlung nicht in die Vorrichtung eingelegt ist, d.h. der erste oder dritte Betriebszustand detektiert wird. Die Signale können auch von der zentralen Steuer- und Recheneinheit 13 der Blutbehandlungsvorrichtung empfangen werden, die in Abhängigkeit von dem Betriebszustand Steuersignale erzeugt. Beispielsweise kann die Steuer- und Recheneinheit 13 derart konfiguriert sein, dass ein Steuersignal für einen Eingriff in die Maschinensteuerung erzeugt wird, wenn die Steuer- und Recheneinheit das Signal für den zweiten Betriebszustand (Fig. 7) empfängt. Beispielsweise kann die Steuer- und Recheneinheit 13 die Blutbehandlung erst dann freigeben, wenn das Signal für den zweiten Betriebszustand (Fig. 7) empfangen wird, d.h. die Blutbehandlung nicht freigeben, wenn Signal für den ersten und dritten Betriebszustand (Fig. 6 und Fig. 8) empfangen wird.

## Patentansprüche

1. Vorrichtung zum Abklemmen einer Schlauchleitung, die in die Vorrichtung eingelegt wird, mit
einem Klemmkörper (19), der in eine die Schlauchleitung abklemmende Position und eine die Schlauchleitung freigebende Position bewegbar ist,
einer elektromagnetischen Betätigungseinheit (20) für den Klemmkörper (19), die eine Spule (21) zur Erzeugung eines Magnetfeldes aufweist, das eine Kraft auf den Klemmkörper (19) ausübt, so dass der Klemmkörper bewegbar ist,
und einer die Position des Klemmkörpers (19) überwachenden Überwachungseinrichtung (23),
wobei
die Überwachungseinrichtung (23) eine Messeinrichtung (24) zum Messen einer von der Stellung des Klemmkörpers (19) abhängigen elektrischen Eigenschaft der Spule (21) und eine Auswerteeinrichtung (25) zum Bestimmen der Position des Klemmkörpers (19) auf der Grundlage der elektrischen Eigenschaft der Spule aufweist, wobei
die Überwachungseinrichtung (23) eine Auswerteeinrichtung (25) aufweist, die eine Vergleichseinrichtung (26) aufweist, die derart ausgebildet ist, dass die elektrische Eigenschaft der Spule (26) mit mindestens einem vorgegebenen Grenzwert verglichen wird, wobei die Auswerteeinrichtung (25) derart ausgebildet ist, dass bei Überschreiten oder Unterschreiten des mindestens einen Grenzwertes auf die von dem Klemmkörper (19) eingenommene Position geschlossen wird, **dadurch gekennzeichnet, dass** die Vergleichseinrichtung derart ausgebildet ist, dass
auf eine erste Position geschlossen wird, in der der Klemmkörper (19) eine vollständig geöffnete Position einnimmt, in der eine Schlauchleitung in die Vorrichtung zum Abklemmen der Schlauchleitung eingelegt werden kann, wenn die elektrische Eigenschaft der Spule (21) kleiner als ein erster oberer Grenzwert und/oder größer als ein erster unterer Grenzwert ist,
auf eine zweite Position geschlossen wird, in der der Klemmkörper (19) eine Position einnimmt, in der eine in die Vorrichtung zum Abklemmen einer Schlauchleitung eingelegte Schlauchleitung abgeklemmt wird, wenn die elektrische Eigenschaft der Spule (21) kleiner als ein zweiter oberer Grenzwert und/oder größer als ein zweiter unterer Grenzwert ist, und
auf eine dritte Position geschlossen wird, in der der Klemmkörper (19) eine vollständig geschlossene Position einnimmt, in der eine Schlauchleitung in die Vorrichtung zum Abklemmen einer Schlauchleitung nicht eingelegt ist, wenn die elektrische Eigenschaft der Spule (21) kleiner als ein dritter oberer Grenzwert und/oder größer als ein dritter unterer Grenzwert ist, und
die Auswerteeinrichtung (25) derart ausgebildet ist, dass sie in Abhängigkeit von der ermittelten Position
ein Signal erzeugt, das einem jeweiligen Betriebszustand zugeordnet ist.

2. Vorrichtung nach Anspruch 1, **dadurch gekennzeichnet, dass** der Klemmkörper (19) als ein axial beweglicher Anker ausgebildet ist und die Spule (21) eine Hohlspule ist, in die der Klemmkörper eintaucht.

3. Vorrichtung nach Anspruch 1 oder 2, **dadurch gekennzeichnet, dass** der Klemmkörper (19) in eine die Schlauchleitung abklemmende Position federnd vorgespannt ist.

4. Vorrichtung nach einem der Ansprüche 1 bis 3, **dadurch gekennzeichnet, dass** die von der Stellung des Klemmkörpers (19) abhängige elektrische Eigenschaft der Spule (21) die Induktivität L der Spule und die Messeinrichtung (24) eine Messeinrichtung zum Messen der Induktivität L der Spule ist.

5. Vorrichtung nach einem der Ansprüche 1 bis 4, **dadurch gekennzeichnet, dass** die Messeinrichtung (24) eine Spannungsquelle (26) zur Erzeugung einer Testspannung aufweist, die an die Spule (21) angelegt wird, so dass durch die Spule ein Strom fließt, und einen Spannungsmesser (27) aufweist, der die zeitliche Änderung der an der Spule abfallenden Spannung misst, wobei die Auswerteeinrichtung (25) derart ausgebildet ist, dass der zeitliche Verlauf des Spannungsabfalls an der Spule ausgewertet wird.

6. Vorrichtung nach Anspruch 5, **dadurch gekennzeichnet, dass** die Vergleichseinrichtung (26) derart ausgebildet ist, dass die Abnahme der Spannung in einen vorgegebenen Zeitintervall Δt mit mindestens einem Grenzwert verglichen wird.

7. Vorrichtung nach einem der Ansprüche 1 bis 6, **dadurch gekennzeichnet, dass** Überwachungseinrichtung (23) eine Anzeigeeinrichtung (28) aufweist, die derart ausgebildet ist, dass die von dem Klemmkörper (19) eingenommene Position angezeigt wird.

8. Vorrichtung nach einem der Ansprüche 1 bis 7, **dadurch gekennzeichnet, dass** Überwachungseinrichtung (23) eine Alarmeinrichtung (29) aufweist, die derart ausgebildet ist, dass ein Alarm gegeben wird, wenn der Klemmkörper (19) eine bestimmte Position einnimmt.

9. Medizinische Behandlungsvorrichtung, insbesondere Vorrichtung zur extrakorporalen Blutbehandlung, mit einer Vorrichtung (14) nach einem der Ansprüche 1 bis 8 zum Abklemmen einer Schlauchleitung, insbesondere der venösen Schlauchleitung (6) des extrakorporalen Blutkreislaufs.

10. Verfahren zur Überwachung einer Vorrichtung zum Abklemmen einer Schlauchleitung, welche einen Klemmkörper, der in eine die Schlauchleitung abklemmende Position und eine die Schlauchleitung freigebende Position bewegbar ist, und eine elektromagnetische Betätigungseinheit für den Klemmkörper aufweist, welche eine Spule zur Erzeugung eines Magnetfeldes aufweist, das eine Kraft auf den Klemmkörper ausübt, so dass der Klemmkörper bewegt wird,
wobei
eine von der Stellung des Klemmkörpers abhängige elektrischen Eigenschaft der Spule gemessen und auf der Grundlage der elektrischen Eigenschaft der Spule auf die Stellung des Klemmkörpers geschlossen wird, **dadurch gekennzeichnet,**
**dass**
auf eine erste Position geschlossen wird, in der der Klemmkörper eine vollständig geöffnete Position einnimmt, in der eine Schlauchleitung in die Vorrichtung zum Abklemmen der Schlauchleitung eingelegt werden kann, wenn die elektrische Eigenschaft der Spule kleiner als ein erster oberer Grenzwert und/oder größer als ein erster unterer Grenzwert ist,
auf eine zweite Position geschlossen wird, in der der Klemmkörper eine Position einnimmt, in der eine in die Vorrichtung zum Abklemmen des Ventilkörpers eingelegte Schlauchleitung abgeklemmt wird, wenn die elektrische Eigenschaft der Spule kleiner als ein zweiter oberer Grenzwert und/oder größer als ein zweiter unterer Grenzwert ist, und
auf eine dritte Position geschlossen wird, in der der Klemmkörper eine vollständig geschlossene Position einnimmt, in der eine Schlauchleitung in die Vorrichtung zum Abklemmen des Ventilkörpers nicht eingelegt ist, wenn die elektrische Eigenschaft der Spule kleiner als ein dritter oberer Grenzwert und/oder größer als ein dritter unterer Grenzwert ist,
wobei in Abhängigkeit von der ermittelten Position ein Signal erzeugt wird, das einem jeweiligen Betriebszustand zugeordnet ist.

11. Verfahren nach Anspruch 10, **dadurch gekennzeichnet, dass** die von der Stellung des Klemmkörpers abhängige elektrische Eigenschaft der Spule die Induktivität L der Spule ist.

12. Verfahren nach Anspruch 10 oder 11, **dadurch gekennzeichnet, dass** die elektrische Eigenschaft der Spule mit mindestens einem vorgegebenen Grenzwert verglichen wird, wobei bei Überschreiten oder Unterschreiten des mindestens einen Grenzwertes auf die von dem Klemmkörper eingenommene Position geschlossen wird.

13. Verfahren nach einem der Ansprüche 10 bis 12, **dadurch gekennzeichnet, dass** an die Spule eine Testspannung angelegt wird, so dass durch die Spule ein Strom fließt, und die zeitliche Änderung der an der Spule abfallenden Spannung gemessen wird, wobei der zeitliche Verlauf des Spannungsabfalls an der Spule ausgewertet wird.

## Claims

1. Device for clamping a hose line which is inserted into the device, comprising
a clamping body (19) which can be moved into a position in which it clamps the hose line and into a position in which it releases the hose line,
an electromagnetic actuation unit (20) for the clamping body (19), comprising a coil (21) for generating a magnetic field which exerts a force on the clamping body (19) such that said clamping body is movable,
and a monitoring device (23) which monitors the position of the clamping body (19),
the monitoring device (23) comprising a measurement device (24) for measuring an electrical property of the coil (21) that is dependent on the position of the clamping body (19), and an evaluation device (25) for determining the position of the clamping body (19) on the basis of the electrical property of the coil,
the monitoring device (23) comprising an evaluation device (25) which comprises a comparison device (26) which is designed such that the electrical property of the coil (26) is compared with at least one specified threshold value, the evaluation device (25) being designed such that, when the electrical property exceeds or falls below the at least one threshold value, a conclusion is made regarding the position assumed by the clamping body (19),
**characterised in that** the comparison device is designed such that,
if the electrical property of the coil (21) is below a first upper threshold value and/or above a first lower threshold value, it is concluded that a first position has been assumed in which the clamping body (19) assumes a completely open position in which a hose line can be inserted into the device for clamping the hose line,
if the electrical property of the coil (21) is below a second upper threshold value and/or above a second lower threshold value, it is concluded that a second position has been assumed in which the clamping body (19) assumes a position in which a hose line inserted into the device for clamping a hose line is clamped, and
if the electrical property of the coil (21) is below a third upper threshold value and/or above a third lower threshold value, it is concluded that a third position has been assumed in which the clamping body (19) assumes a completely closed position in which a hose line is not inserted into the device for clamping a hose line, and
the evaluation device (25) is designed such that the evaluation device, depending on the detected position, generates a signal assigned to a relevant operating state.

2. Device according to claim 1, **characterised in that** the clamping body (19) is designed as an axially movable anchor, and the coil (21) is a hollow coil which the clamping body penetrates.

3. Device according to either claim 1 or claim 2, **characterised in that** the clamping body (19) is resiliently biased into a position in which it clamps the hose line.

4. Device according to any of claims 1 to 3, **characterised in that** the electrical property of the coil (21) that is dependent on the position of the clamping body (19) is the inductance L of the coil, and the measurement device (24) is a measurement device for measuring the inductance L of the coil.

5. Device according to any of claims 1 to 4, **characterised in that** the measurement device (24) comprises a voltage source (26) for generating a test voltage which is applied to the coil (21) such that a current flows through the coil, and a voltmeter (27) which measures the change over time in the voltage which drops across the coil, the evaluation device (25) being designed such that the profile over time of the voltage drop across the coil is evaluated.

6. Device according to claim 5, **characterised in that** the comparison device (26) is designed such that the decrease in voltage in a specified time period Δt is compared with at least one threshold value.

7. Device according to any of claims 1 to 6, **characterised in that** the monitoring device (23) comprises a display device (28) which is designed such that the position assumed by the clamping body (19) is displayed.

8. Device according to any of claims 1 to 7, **characterised in that** the monitoring device (23) comprises an alarm device (29) which is designed such that an alarm is emitted if the clamping body (19) assumes a particular position.

9. Medical treatment device, in particular a device for extracorporeal blood treatment, comprising a device (14) according to any of claims 1 to 8 for clamping a hose line, in particular the venous hose line (6) of the extracorporeal blood circuit.

10. Method for monitoring a device for clamping a hose line, which device comprises a clamping body, which can be moved into a position in which it clamps the hose line and into a position in which it releases the hose line, and an electromagnetic actuation unit for the clamping body, which actuation unit comprises a coil for generating a magnetic field which exerts a force on the clamping body such that the clamping body moves,
an electrical property of the coil that dependent on the position of the clamping body being measured and a conclusion is made regarding the position of the clamping body on the basis of the electrical property of the coil,
**characterized in that**,
if the electrical property of the coil is below a first upper threshold value and/or above a first lower threshold value, it is concluded that a first position has been assumed in which the clamping body assumes a completely open position in which a hose line can be inserted into the device for clamping the hose line,
if the electrical property of the coil is below a second upper threshold value and/or above a second lower threshold value, it is concluded that a second position has been assumed in which the clamping body assumes a position in which a hose line inserted into the device for clamping the valve body is clamped, and
if the electrical property of the coil is below a third upper threshold value and/or above a third lower threshold value, it is concluded that a third position has been assumed in which the clamping body assumes a completely closed position in which a hose line is not inserted into the device for clamping the hose line,
a signal being generated depending on the detected position, which is assigned to a relevant operating state.

11. Method according to claim 10, **characterised in that** the electrical property of the coil that is dependent on the position of the clamping body is the inductance L of the coil.

12. Method according to either claim 10 or claim 11, **characterised in that** the electrical property of the coil is compared with at least one specified threshold value, a conclusion being made regarding the position assumed by the clamping body, when the electrical property exceeds or falls below the at least one threshold value.

13. Method according to any of claims 10 to 12, **characterised in that** a test voltage is applied to the coil such that a current flows through the coil and the change over time in the voltage which drops across the coil is measured, the profile over time of the voltage drop across the coil being evaluated.

## Revendications

1. Dispositif pour clamper une tubulure qui est placée dans le dispositif, avec un corps de clampage (19) qui peut être déplacé dans une position de clampage de la tubulure et dans une position de déblocage de la tubulure,
une unité d'actionnement (20) électromagnétique pour le corps de clampage (19), qui présente une bobine (21) pour générer un champ magnétique, qui exerce une force sur le corps de clampage (19) de sorte que le corps de clampage peut être déplacé,
et un système de surveillance (23) surveillant la position du corps de clampage (19),
dans lequel
le système de surveillance (23) présente un système de mesure (24) pour mesurer une propriété électrique, dépendant de la position du corps de clampage (19), de la bobine (21) et un système d'évaluation (25) pour définir la position du corps de clampage (19) sur la base de la propriété électrique de la bobine,
dans lequel le système de surveillance (23) présente un système d'évaluation (25) qui présente un système de comparaison (26) réalisé de telle manière que la propriété électrique de la bobine (26) est comparée à au moins une valeur limite prédéfinie, le système d'évaluation (25) étant réalisé de telle manière que lors du dépassement ou du non-dépassement de l'au moins une valeur limite, la position adoptée par le corps de clampage (19) est déduite,
**caractérisé en ce que** le système de comparaison est réalisé de telle sorte qu'est déduite une première position dans laquelle le corps de clampage (19) adopte une position totalement ouverte dans laquelle une tubulure peut être placée dans le dispositif pour clamper la tubulure quand la propriété électrique de la bobine (21) est inférieure à une première valeur limite supérieure et/ou est supérieure à une première valeur limite inférieure,
est déduite une deuxième position, dans laquelle le corps de clampage (19) adopte une position, dans laquelle une tubulure placée dans le dispositif pour clamper une tubulure est clampée quand la propriété électrique de la bobine (21) est inférieure à une deuxième valeur limite supérieure et/ou est supérieure à une deuxième valeur limite inférieure, et
est déduite une troisième position, dans laquelle le corps de clampage (19) adopte une position totalement fermée, dans laquelle une tubulure n'est pas placée dans le dispositif pour clamper une tubulure quand la propriété électrique de la bobine (21) est inférieure à une troisième valeur limite supérieure et/ou est supérieure à une troisième valeur limite inférieure, et
le système d'évaluation (25) est réalisé de telle manière qu'il génère, en fonction de la position déterminée, un signal qui est associé à un état de fonctionnement respectif.

2. Dispositif selon la revendication 1, **caractérisé en ce que** le corps de clampage (19) est réalisé en tant qu'un ancrage axialement mobile et la bobine (21) est une bobine creuse, dans laquelle le corps de clampage plonge.

3. Dispositif selon la revendication 1 ou 2, **caractérisé en ce que** le corps de clampage (19) est précontraint de manière élastique dans une position de clampage de la tubulure.

4. Dispositif selon l'une quelconque des revendications 1 à 3, **caractérisé en ce que** la propriété électrique de la bobine (21), dépendant de la position du corps de clampage (19), est l'inductance L de la bobine et le système de mesure (24) est un système de mesure pour mesurer l'inductance L de la bobine.

5. Dispositif selon l'une quelconque des revendications 1 à 4, **caractérisé en ce que** le système de mesure (24) présente une source de tension (26) pour générer une tension test qui est appliquée sur la bobine (21) de sorte qu'un courant s'écoule à travers la bobine, et présente un voltmètre (27) qui mesure la variation dans le temps de la tension qui chute sur la bobine, le système d'analyse (25) étant réalisé de telle manière que l'évolution dans le temps de la chute de tension sur la bobine est évaluée.

6. Dispositif selon la revendication 5, **caractérisé en ce que** le système de comparaison (26) est réalisé de telle manière que la baisse de la tension dans un intervalle de temps Δt prédéfini est comparée à au moins une valeur limite.

7. Dispositif selon l'une quelconque des revendications 1 à 6, **caractérisé en ce que** le système de surveillance (23) présente un système d'affichage (28) qui est réalisé de telle manière que la position adoptée par le corps de clampage (19) est affichée.

8. Dispositif selon l'une quelconque des revendications 1 à 7, **caractérisé en ce que** le système de surveillance (23) présente un système d'alarme (29) qui est réalisé de telle manière qu'une alarme est émise quand le corps de clampage (19) adopte une certaine position.

9. Dispositif de traitement médical, en particulier dispositif de traitement du sang extracorporel, avec un dispositif (14) selon l'une quelconque des revendications 1 à 8 pour clamper une tubulure, en particulier la tubulure veineuse (6) du circuit sanguin extracorporel.

10. Procédé pour surveiller un dispositif pour clamper une tubulure, lequel présente un corps de clampage, qui peut être déplacé dans une position de clampage de la tubulure et dans une position de déblocage de la tubulure, et une unité d'actionnement électromagnétique pour le corps de clampage, laquelle présente une bobine pour générer un champ magnétique, qui exerce une force sur le corps de clampage de sorte que le corps de clampage est déplacé,
dans lequel
une propriété électrique, dépendant de la position du corps de clampage, de la bobine est mesurée et la position du corps de clampage est déduite sur la base de la propriété électrique de la bobine,
**caractérisé en ce qu'**est déduite une première position, dans laquelle le corps de clampage adopte une position totalement ouverte dans laquelle une tubulure peut être placée dans le dispositif pour clamper la tubulure quand la propriété électrique de la bobine est inférieure à une première valeur limite supérieure et/ou est supérieure à une première valeur limite inférieure,
est déduite une deuxième position, dans laquelle le corps de clampage adopte une position dans laquelle une tubulure placée dans le dispositif pour clamper le corps de soupape est clampée quand la propriété électrique de la bobine est inférieure à une deuxième valeur limite supérieure et/ou est supérieure à une deuxième valeur limite inférieure, et
est déduite une troisième position, dans laquelle le corps de clampage adopte une position totalement fermée, dans laquelle une tubulure n'est pas placée dans le dispositif pour clamper le corps de soupape quand la propriété électrique de la bobine est inférieure à une troisième valeur limite supérieure et/ou est supérieure à une troisième valeur limite inférieure,
dans lequel est généré, en fonction de la position déterminée, un signal qui est associé à un état de fonctionnement respectif.

11. Procédé selon la revendication 10, **caractérisé en ce que** la propriété électrique, dépendant de la position du corps de clampage, de la bobine est l'inductance L de la bobine.

12. Procédé selon la revendication 10 ou 11, **caractérisé en ce que** la propriété électrique de la bobine est comparée à au moins une valeur limite prédéfinie, la position adoptée par le corps de clampage étant déduite en cas de dépassement ou de non-dépassement de l'au moins une valeur limite.

13. Procédé selon l'une quelconque des revendications 10 à 12, **caractérisé en ce qu'**est appliquée sur la bobine une tension test de sorte qu'un courant s'écoule à travers la bobine et la variation dans le temps de la tension chutant sur la bobine est mesurée, l'évolution dans le temps de la chute de tension sur la bobine étant évaluée.
